# EUROPEAN PATENT APPLICATION

(11) **EP 3 912 477 A1**
(43) Date of publication of application: **24.11.2021**
(21) Application number: 20382418.0
(22) Date of filing: 18.05.2020
(51) Int. Cl.: A23K 50/00, G06Q 10/04, G16H 20/60

(54) **METHOD FOR DETERMINING AN OPTIMIZED GROUP FEED COMPOSITION, CORRESPONDING COMPUTER PROGRAM, LOCAL AND FEDERATED SYSTEMS**

(71) Applicant: Dairy Information Technology Services S.L, 31500 Navarra (ES)
(72) Inventor: Bach, Alejandro, 31500 Tudela (ES); Ahedo, José, 31500 Tudela (ES)
(74) Representative: Curell Suñol S.L.P.

(57) **Abstract**

Method for determining an optimized group feed composition, corresponding computer program, local and federated systems. A daily optimized group feed composition (1) to be fed to a group (2) of animals that are bred to produce milk is determined by:
a) determining a target criterion (3) for optimization, depending on at least one milk parameter;
b) for each animal, determining an optimized animal feed composition (4) as the one that optimizes said target criterion (3) using a local milk prediction model (5); and
c) determining said optimized group feed composition (1) as the aggregation of said optimized animal feed compositions (4);
wherein said local milk prediction model (5) is configured for obtaining a prediction of milk production (52) of one animal in one day depending on input data (51); said input data (51) comprising a feed composition fed to said animal in said day; said prediction of milk production (52) comprising said at least one milk parameter.

## Description

### Field of the invention

The invention lies in the field of dairy industry and the optimization of the production of milk from animals bred to produce milk.

In particular, the invention relates to a method for determining a feed composition to be fed in one day to a group of animals that are bred to produce milk, in particular dairy cattle, sheep or goats, said group having one or more animals.

The invention also relates to a corresponding computer program for implementing said method and to corresponding local and federated systems.

### Prior art

Nutritional models for animals like dairy cattle are known in the art. Said know models calculate nutrient requirements for an animal in order to obtain a specific quantity of milk, using pre-determined algorithms that take into account different parameters of the animal itself, including the energy needed for maintaining the body temperature.

Farmers have been using said nutritional models in order to obtaining the maximum amount of milk for a minimum cost of ingredients. First a target quantity of milk is determined. This can be done according to the experience of the farmer or by giving the animal plenty of feed and observing how much milk it produces. Afterwards, a target quantity of milk is determined, usually a bit over the normal production. For example, the farmer observes that a particular animal produces 40 litres of milk in one day and determines the target quantity to 42 litres. Using that target quantity, the nutritional model is used for determining the minimum amount of nutrients that have to be fed to the animal in order to obtain said target quantity. Using as constraints said minimum amount of nutrients and the maximum that the animal can eat in one day, the known methods use Linear Programming in order to obtain a feed composition having at least said minimum amount of nutrients with a maximum of quantity of feed, not exceeding what the animal is able to eat, and minimizing the cost. For the sake of clarity, the method has been described for only one animal, nevertheless, the usual application is for a group of animals. In the latter case, the farmer calculates the food required for all of the animals in the group using the target quantity of milk for each animal.

Therefore, using the available methods in the art and the aforementioned nutritional models, it is possible to determine the feed to be given to an animal in order to obtain a target production of milk while minimizing the cost, the feed being a list of different ingredients like corn silage, alfalfa hay, barley, etc. and their quantities. It is usual that the different animals in a herd are grouped by pens. Using the aforementioned nutritional models, the solutions that are known in the art calculate the total feed intake required for all the animals of the group, so the farmer can prepare a feed mix containing the ingredients determined by the nutritional model.

Nevertheless, these nutritional models are a rough estimation of the real situation of the animals in a farm. For example, many of the nutritional models assume that each animal has *ad libitum* access to feed and water, and that they are kept under dry and clean conditions. Therefore, these models disregard any competition for the food among the different animals in the group. Environmental influence is sometimes disregarded too, even if some nutritional models further incorporate correcting factors considering the ambient temperature, humidity or housing conditions.

In general, the methods known in the art have other limitations for the dairy industry. One of the limitations is that they assume that all animals behave the same way, regardless of factors such are genetics and farm management, that can also be related with stress levels. Another important limitation is that they disregard many types of nutrients, as well as the possible local differences in the nutrient content of each ingredient. Moreover, the models also disregard non-nutritional factors in the feed composition, for example, the palatability, synergistic or antagonist combinations of ingredients and/or nutrients, etc.

In addition, since the nutritional models are aimed for productivity and/or cost minimization, it is not easy or even possible to use a different criterion. For example, calculating a feed composition that maximizes not the cost of the milk production but benefit obtained from said milk considering the cost of the ingredients and the selling price of the milk, said cost of the milk being often dependent on the milk quality and, in particular, dependent on the nutrients present in the milk.

Therefore, it is necessary to provide a method for determining feed compositions for animals bred to produce milk that allows different criteria and provide a better adaptation for real-life conditions.

### Summary of the invention

The invention is aimed to provide method for determining a feed composition of the type stated at the beginning, being able to avoid the problems that have been identified above.

This purpose is achieved by a method for determining a feed composition of the type indicated at the beginning, characterized in that said method comprises the steps of:
a) determining a target criterion for optimization, said target criterion depending on at least one milk parameter; and
b) for each animal of said group of animals, determining an optimized feed composition as the feed composition that optimizes said target criterion using a local milk prediction model;
c) determining said feed composition to be fed to said group of animals as the aggregation of said optimized feed compositions for each of the animals of said group;
wherein said local milk prediction model is configured for obtaining a prediction of milk production of one animal in one day depending on input data; said input data comprising a feed composition fed to said animal in said day; said prediction of milk production comprising milk output data comprising said at least one milk parameter.

The method can be executed periodically, for example daily, thus obtaining daily optimized group feed compositions. In addition, it can also be executed for different groups of animals, thus obtaining optimized group feed compositions for different groups of animals, or for an entire herd in a farm or a group of farms.

The method of the invention uses a model that is not directed to determine a feed composition for obtaining a predeterminate amount of milk production. Instead, the method uses a predictive model of the expected milk production given a set of input data parameters comprising, at least, a feed composition. In fact, the milk prediction model can be interpreted as a function having an n-dimensional input and m-dimensional output. In this interpretation the n-dimensional input corresponds to the different parameters of the input data including, at least, the feed composition fed to the animal, and the m-dimensional output includes said at least one milk parameter. If only one milk parameter is obtained as output, the function output will be one-dimensional, if two milk parameters are obtained, it will be bi-dimensional, etc. Equivalent mathematical interpretations are possible as it would be evident for those skilled in the art. Therefore, by feeding the model different input data values, it is possible to obtain, as an output, the resulting milk parameters predicted for said input data, and afterwards using said results and input data for the optimization according to different target criteria. Several mathematical optimization methods can be envisaged according to the art.

Thus, this reverse approximation to the problem allows being able to use the same model for different criteria. Indeed, in the known nutritional models, the possible target criterion is only meeting a target milk production, and maybe minimizing the cost. For the method of the invention, different target criteria can be determined. Each target criterion can be simple, i.e. having just one parameter like the quantity of milk produced by the animal, or complex, i.e. having multiple parameters, for example, including the cost of the ingredients, the expected protein content in the milk, etc. Those skilled in the art will understand that step b) can be performed in different ways according to different optimization methods for one variable or multi-variable optimization. Preferably, said target criterion is defined by a cost function and said step b) comprises a mathematical minimization of said cost function using said predicted milk parameters predicted for said input data.

The optimized feed composition is thereby determined for each animal of the group. In the case that the group has more than one animal, it is considered that all the animals share the same feed and eat from the same place. In this case the feed composition is the aggregation of the optimized feed composition determined for each of the animals in the group. In the case that the group contains only one animal, it is considered that the feed composition will be given only to that animal, so the aggregation only contains one optimized feed composition. For example, in a farm having a herd of animals comprising different pens, each pen composed of several animals, the method can be applied pen by pen, in the case that all the animals in the pen fed together, or animal by animal, in the case that each animal is fed independently. The skilled person will understand that, in the former case, it is preferred that all the animals in the pen have similar characteristics. In a preferred embodiment, the same local milk prediction model is used for all the herd in a farm, irrespective of the groups of animals. In an alternative embodiment, a local milk prediction model is aimed for a single group of animals.

Preferably, said feed composition comprises a list of quantities of ingredients, a list of quantities of nutrients or a combination thereof, i.e. a list of one or more ingredients and their quantities, a list of one or more nutrients and their quantities or a combination thereof. Depending on the ingredient or nutrient, the quantity can be, for example a weight, volume or a percentage thereof. Therefore, the resulting list of ingredients and/or nutrients can be used in the farm for creating a feed mix to be fed to the group of animals. In the cases where the feed composition comprises ingredients, the model is adapted for predicting the milk parameters as a function of the ingredients. The latter allows to adapt the method to local variations in the nutrient compositions and availability of each ingredient.

In a preferred embodiment, determining an optimized feed composition comprises a first optimization step using said local milk prediction model, and a second optimization step using further constraints. This embodiment is particularly advantageous for complex target criteria, because it allows to divide the optimization in a part that relates to the milk prediction model, and a part related to other constrains or parameters. By the way of a non-limiting example, in the case that the milk prediction model can be used for obtaining a the predicted milk production based on a list of nutrients and their quantities, this embodiment allows to, first, obtaining a list of nutrients that maximize the milk production for each animal and, second, obtaining a list of the ingredients that have said required nutrients but that minimize the cost. In this example, the first optimization step is performed using the local milk prediction model, and the second optimization step is performed using further constraints that relate to the available ingredients, their nutritional contents and cost.

Preferably, said at least one milk parameter comprises at least one of the following milk parameters:
- quantity of milk, thus allowing target criteria like maximizing the production of milk;
- protein content of the milk;
- fat content of the milk;
- lactose content of the milk;
- calcium content of milk; and
- fatty acid profile of milk;
or combinations thereof. The parameters relating to milk contents are particularly advantageous for target criteria that include quality-related information of the milk.

Preferably, said input data of said local milk prediction model further comprises at least one animal parameter, preferably, at least one of the list consisting in:
- body weight;
- stage of lactation;
- stage of pregnancy;
- lactation number; and
- genomic value of the animal.
Therefore, the model is able to make a prediction of the milk, not only based in feed intake but also in other parameters that have been found relevant for the production of milk.

Preferably, said input data of said local milk prediction model further comprises at least one environmental parameter, preferably at least one of temperature, humidity and hours of exposure to daylight, thus accounting the influence of the environment in the production of milk.

Preferably, said target criterion is one of:
- maximizing milk production; thus being able to determine feed compositions that lead to a maximum of milk production by the animals, preferably further minimizing the production cost;
- obtaining a predetermined milk protein content value or range; thus being able to produce milk with a quality criterion based on the protein content;
- obtaining a predetermined milk fat content value or range; thus being able to produce milk with a quality criterion based on the fat content; and
- maximizing milk production benefit.
In order to maximize the benefit, it is necessary that the target criterion includes not only the milk production but also the milk selling price and the price of the ingredients to be used in the feed composition. In particular, the price of the milk often depends on several parameters, for example, the quantity of milk sold, and quality parameters of the milk. Therefore, it is also possible that the target criterion is a combination of the criteria defined above, which is particularly advantageous if the target criterion comprises not only quantity or benefit, but also quality parameters of the milk. This complexity and variety in the target criteria is possible due to the fact that the model used is not itself aimed for determining the feed composition, instead, the milk prediction model is aimed for predicting milk parameters given a set of input data, and the feed composition is determined using an optimization according to the inputs and outputs of the model.

Preferably, the method further comprises the steps of:
d) for each animal of the group, receiving a set of measures, comprising:
   - a measure of consumed feed, said consumed feed being the feed composition that has been consumed by said animal in one day;
   - a measure of milk production, said milk production being the milk that has been produced by said animal in said day, said measure of milk production comprising said at least one milk parameter;
   - preferably, at least a measure of an animal parameter; and
   - preferably, at least a measure of an environmental parameter;
e) updating said local milk prediction model by incorporating said set of measures for each animal of the group.

Therefore, the model is updated based on a set of measures corresponding to input data of the model (feed intake and preferably other parameters relating to the animal and/or the environment) and to the output data of the model (the parameter or parameters related to the milk production). Preferred examples of the animal and environmental parameters have been disclosed above. Even if the predictions are accurate, the real measures can differ, therefore, updating the model according to the measures tends to improve its results. Moreover, this updating process results in a model that can, potentially, take into account local variations or complex dependencies in the milk production, for example, those relating to the nutrient profiles of the ingredients, the metabolic profiles of the animals, the environmental effects, management effects such as stocking density, etc.

Preferably, the steps d) and e) are repeated daily, so that the milk prediction model is updated with according to the interval of time of reference, i.e. the model predicts the milk production in one day according to the input data in that day. Preferably, the method further comprises an initial learning phase, wherein only steps d) and e) are repeated daily, and a production phase comprising steps a) to e).

Preferably, said measures of consumed feed and said measures of milk production are, independently, obtained using respective moving averages, in order to filter transitory results or brief variations. Moving averages, also referred as rolling averages, are thus used in order to obtain representative data having less variance. In a preferred embodiment, the window for the average is determined according to the variance or the standard deviation of the sample, so that, if the variance is high, a long average window is selected, preferably, up to ten days, while, if the variance is low, a short average window is selected, the minimum being one day. Preferably, the length of the average window further depends on the size of the group, so that, for groups having a large number of animals, the average windows is shorter than for groups having a small number of animals.

Preferably, for each animal of the group, said measure of consumed feed is an estimated measure based in a measure of feed consumed by all the animals in the group. This is particularly advantageous in the case that all the animals of the group feed from the same feeding point, when it is not possible to measure the feed intake of each animal independently. Different estimations can be envisaged, by the way of a non-limiting example, simply dividing the feed consumed by all the animals by the number of animals in the group.

In a preferred embodiment, said estimated measures are obtained by:
- obtaining an expected consumption for each animal of the group;
- obtaining a total expected consumption for the group, being the aggregation of each expected consumption for each of the animals of the group;
- dividing said measure of feed consumed by all the animals in the group by said total expected consumption for the group, thus obtaining a correction factor; and
- for each animal in the group applying said correction factor to said expected consumption for said animal.
The differences in feed intake among the animals can be significative. As a consequence, estimating that all the animals eat the same can lead to erroneous results. This preferred embodiment allows to minimize the impact of this effect by using particular estimated expected consumptions for each animal, and then normalizing the expected consumptions according to the real measure of total intake using said correction factor.

Other alternatives having equivalent calculation methods can be envisaged. As a non-limitative example, in an alternative embodiment, for each animal of the group, said estimated measure is obtained by applying a consumption factor of said animal to said measure of feed consumed by all the animals in the group; wherein said consumption factors are determined by:
- obtaining an expected consumption for each animal of the group;
- obtaining a total expected consumption for the group, being the aggregation of each expected consumption for each of the animals of the group; and
- for each animal in the group determining said consumption factor as the ratio between said expected consumption for said animal and said total expected consumption.
In this case, different consumption factors are obtained for each animal, and then said factors are used in regards to the measure of the feed consumed by all the animals in the group.

The factor for each animal is obtained in a previous step as the ratio between the feed expected to be consumed by each animal and the total feed expected to be consumed. For each animal an expected consumption is obtained, for example, using a model of feed intake, using previous observations of feed intake by said animal, etc. The total expected consumption corresponds to the addition of the expected consumptions of all the animals. Therefore, the factor for each animal is related to the portion of the total expected feed consumption that it is expected to be consumed by said animal. According to this definition, the sum of all the factors for all the animals in the group is 1. Nevertheless, other equivalent relationships can be envisaged, for example, using percentages instead of factors or other weighted relations. Even if this embodiment is still an approximation, it leads to more accurate results, in special if the expected consumptions are close to the reality.

Preferably, said expected consumption for each animal is obtained by using the optimized feed composition for said animal, determined in step b), thus using the milk prediction model in order to estimate the consumption. This option is particularly advantageous when the model is well trained.

In an alternative embodiment, said expected consumption for each animal is obtained by using a prediction model configured to estimate the feed intake of one animal based on its milk production and/or animal characteristics. Since the prediction model for the estimation is not the same than the local milk prediction model, this embodiment is less likely to show positive feedbacks in the adaptation, and is, therefore, particularly suitable for the initial updating iterations when the milk prediction model is not fully trained. Several prediction models known in the art can be envisaged, by the way of a non-limiting example, the NRC 2001 by the National Research Council regarding Nutrient Requirements of Dairy Cattle.

Preferably, said local milk prediction model is a neural network, thus being able to adapt to the updates based on measures, with a limited a priori considerations. More preferably, said neural network is a Long-Short Term Memory, LSTM neural network. In LSTM neural networks each node has a feedback, therefore they show a memory effect that keeps the variations and improvements during some time. This effect makes the particularly well suited for the type of usage in the milk prediction model.

Preferably, the method further comprises:
- sending to a central module:
   - said set of measures; and
   - the internal status of the neural network nodes of said local milk prediction model;
- receiving from said central module an updated milk prediction model; and
- replacing the current local milk prediction model with said updated milk prediction model.
Therefore, the method local milk prediction model can be improved according to data coming from a plurality of other local milk prediction models. Said central module is preferably one or more servers that can be accessed remotely, for example, using internet as the communication platform.

Preferably, said updated milk prediction model is generated using federated learning which is a known strategy of distributed learning used in the field of the neural networks. In the method of the invention, it has been found that this kind of learning strategy is particularly advantageous in order to take advantage of the information coming from multiple sources (i.e. different local milk prediction models), but at the same time, maintain a local adaptation that can take into consideration local particularities for each farm.

The invention also refers to a computer program containing program instruction codes that, when executed by a computer, lead to said computer to perform the method described above.

The invention also refers to a local system for determining feed compositions to be fed to at least one group of animals that are bred to produce milk, in particular dairy cattle, sheep or goats, each group of animals having one or more animals, characterized in that said system comprises a control module comprising a local milk prediction model and is configured for performing the method according to any of the embodiments disclosed above for each group of animals, thus having the same technical effects and advantages that have already been explained.

Preferably, said local system further comprises a mobile device to assist in the creation of a feed mix that will be given to one group of animals of said at least one group of animals, said mobile device comprising:
- display means, for displaying information to a user;
- communication means, for communication with said control module; and
- weight measure receiving means, for receiving measures of weight of ingredients;
wherein said mobile device is configured for:
- receiving from said control module a feed composition for one group of animals of said at least one group of animals, said feed composition comprising a list of ingredients and the weight of each of said ingredients that has be added to a feed mix that will be given to said group of animals;
- for each of said ingredients of said list of ingredients:
   - displaying said weight of said ingredient using said display means; and
   - receiving a measure of the weight of said ingredient that has been added to said feed mix;
- and, sending to said control module said measures of the weight of each ingredient;
wherein said control module is configured for determining a measure of feed consumed by all the animals in said group according to said measures of the weight of each ingredient.

In the dairy farms, it is usual that the animals are fed using a feed mix containing different ingredients, for example, corn silage, alfalfa hay, barley, etc. said ingredients are taken from their respective storage locations, mixed, and then offered to the animals. The feed mix is often transported using trucks or mixing wagons. Thus, the mobile device disclosed herein, assist in the creation of the feed mix using the feed composition that has been determined by the method by displaying the quantities of each ingredient.

Preferably, the device further comprises location means, for determining the location of the mobile device regarding the place of storage of each ingredient. The latter allows displaying of the quantities of each ingredient when the mobile device is located where said ingredient is. Different options can be envisaged for said location means, by the way of non-limiting examples, using GPS, NFC or beacons. Besides, the mobile device is also used for receiving the measures of the actual quantity of each ingredient which are often slightly different from the quantity that has been determined for the feed composition. Using these measures, the control module can determine the real amount of feed offered to the animals that then used for determining the measure of consumed feed, the latter being used for updating the local milk prediction model.

Preferably, said control module is further configured for obtaining a measure of weight of the remaining feed that has not been consumed by all the animals in said group, and wherein determining a measure of feed consumed by all the animals further includes said weight of the remaining feed, thereby adjusting the measure in the case that the animals have not consumed all the feed mix that has been provided to them, thus improving the accuracy in the measures and, consequently, of the updated model.

The invention also refers to a federated system for determining feed compositions to be fed to at least one group of animals that are bred to produce milk, in particular dairy cattle, sheep or goats, each group of animals having one or more animals, characterized in that said system comprises:
- at least one local system as disclosed above; and
- a central module, comprising a seed milk prediction model;
wherein said seed milk prediction model and the local milk prediction models of said at least one local system are all neural networks, preferably LSTM neural networks, having the same node structure; each neural network having an internal status;
said central module configured for receiving from each control module corresponding to each of said at least one local system:
- a set of measures; and
- said internal status of the neural network of said local milk prediction model;
said set of measures comprising, for each animal:
- a measure of consumed feed, said consumed feed being the feed composition that has been consumed by said animal in one day;
- a measure of milk production, said milk production being the milk that has been produced by said animal in said day, said measure of milk production comprising said at least one milk parameter;
- preferably, at least a measure of an animal parameter; and
- preferably, at least a measure of an environmental parameter;
said central module further configured for generating, using federated learning, an updated milk prediction model for each of said at least one local system; and sending each updated milk prediction model to the corresponding control module of each local system.

The federated system disclosed herein is thus able to improve the seed model using the data coming from different local systems. The seed model is therefore able to accumulate correlations between the input data and the milk parameters relying on potentially many sources of information. In addition, each local system can still maintain adaptations related to the local conditions, thanks to the federated learning, thus obtaining more accurate local results.

### Brief description of the figures

Further advantages and features of the invention will become apparent from the following description, in which, without any limiting character, preferred embodiments of the invention are disclosed, in reference to the accompanying figures:
Fig. 1 is a simplified flowchart of the steps for determining a feed composition according to one embodiment of the method of the invention.
Fig. 2 is a simplified flowchart of the inputs and outputs of the local milk prediction model.
Fig. 3 is a representation of the update of the local milk prediction model using a set of measures for different animals.
Figs. 4A, 4B and 4C show a comparison between the real daily milk production (black line), the prediction according to current state of the art (dashed line), and the prediction using the local milk production model according to one embodiment of the invention (grey line). Each graph corresponds to a different animal of a group of animals. The horizontal axis is the day, and the vertical the yield of milk in kg per day.
Fig. 5 is a simplified diagram of an embodiment of the federated system according to the invention.

### Detailed description of embodiments o the invention

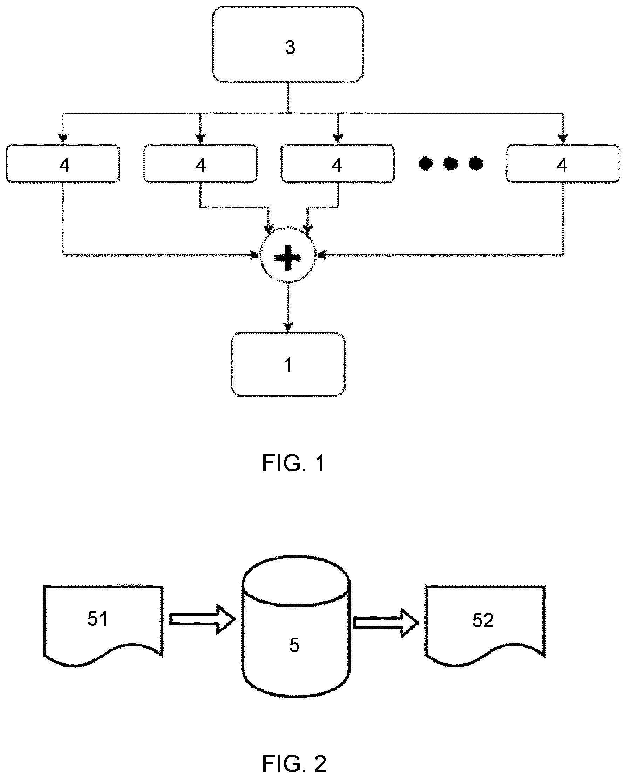

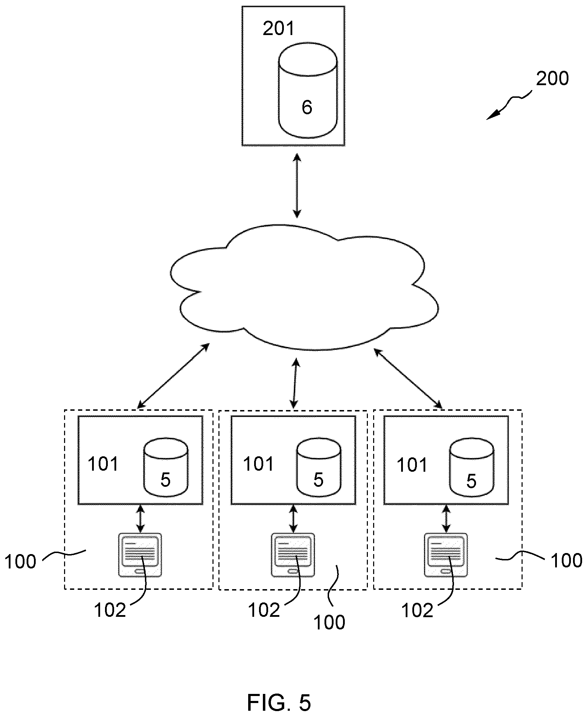

The figures 1 to 4 show one exemplary embodiment of the method for determining an optimized group feed composition 1 to be fed in one day to a group 2 of animals that are bred to produce milk, in particular dairy cattle, sheep or goats, said group 2 having one or more animals. Figure 5 shows a corresponding federated system according to the same embodiment. For this embodiment, the animals are dairy cattle and the group 2 of animals is a pen 2 containing initially 23 animals. The skilled person will understand that these numbers are non-limiting and used just for clarification purposes.

Fig. 1 shows that the method comprises the steps described hereinafter:
a) Determining a target criterion 3 for optimization, said target criterion 3 depending on at least one milk parameter. In the case of the example, the target criterion 3 used for the examples is milk production maximization at minimum cost for the ingredients. The milk parameter is milk production in kilograms. The milk production in one day is often referred as yield milk in the art.
b) For each animal of the group 2 of animals, determining an optimized animal feed composition 4 as the feed composition that optimizes the target criterion 3 using a local milk prediction model 5. The group 2 is a pen containing initially 23 dairy cows. In this first embodiment, each optimized animal feed composition 4 is a list of ingredients and their quantities that have to be given to the corresponding animal in order to optimize the target criterion 3.
c) Determining the optimized group feed composition 1 to be fed to the group 2 of animals as the aggregation of the optimized animal feed compositions 4 for each of the animals of said group 2. For this first embodiment, the optimized group feed composition 1 is a list containing all the ingredients of each of the optimized animal feed compositions 4 and the sum of their quantities for all the animals in the pen 2.
The method of the example is repeated daily, thus obtaining a daily optimized feed composition for the group of animals 2.

The local milk prediction model 5 is configured for obtaining a prediction of milk production 52 of one animal in one day depending on input data 51 as shown in Fig. 2. For the case of the first embodiment, the local milk prediction model 5 is a Long-Short Term Memory, LSTM, neural network, and the input data 51 comprises:
- A feed composition fed to said animal in said day, in this embodiment, said feed composition is the amount of dry feed provided to the cow in kg, the crude protein in kg, the neutral detergent fibre in kg, and the energy of the feed in Mcal.
- The following animal parameters: body weight in kg, stage of lactation in days, and lactation number, the latter being the number of times that the cow has been in lactation.
- The following environmental parameters for said day: minimum temperature, maximum temperature and average temperature. All temperatures measured in Celsius.
The prediction of milk production 52 comprises milk output data comprising said at least one milk parameter, in this embodiment, the kilograms of milk production for said day. Other units can be envisaged for the parameters described above, by the way of an example, using imperial units and/or giving the crude protein content and detergent fibre as percentages of the amount of dry feed.

In order to facilitate the understanding of the method, a real-life example of application of the step b) is given herein for one of the cows in the pen 2 for one day. For this embodiment, determining the optimized animal feed composition 4 comprises a first optimization step using said local milk prediction model 5, and a second optimization step using further constraints. In the first optimization step, the local milk production model 5 is tested with the following input data 51:
- The cow body weight (694kg), stage of lactation (51 days) and lactation number (3) in that day.
- The minimum temperature (19.6°C), maximum temperature (42.7°C) and average temperature (31.8°C) expected for that day.
- A plurality of seven different feed compositions, each feed composition having different values of the amount of dry feed, crude protein, neutral detergent fibre, and the energy of the feed.
Thus obtaining form the local milk production model 5 a plurality of seven predictions of milk production 52 for said cow in said day. Using standard mathematical optimization methods, it is calculated that the maximum production of milk is 53 kg, and that said production would be achieved with a feed composition having 25.2 kg of dry feed containing 14.2% of crude protein and 28.2% of neutral detergent fibre, as well as 1.62 Mcal of net energy.

In the second optimization step, a linear programming is performed using the feed composition obtained in the first optimization step as constraints together with a list of available ingredients containing their nutritional compositions and prices. After this step, the result is the optimal combination of ingredients that satisfy the supply nutritional requirements for the cow at the least possible cost:
- Alfalfa hay: 5.32 kg/d
- Wet brewers grains: 9.0 kg/d
- Orange pulp (fresh): 4.8 kg/d
- Straw: 4.0 kg/d
- Corn silage: 17.0 kg/d
- Mineral premix: 0.5 kg/d
- Corn grain: 3.74 kg/d
- Rapeseed meal: 2.83 kg/d
- Wheat grain: 1.5 kg/d
- Cookie waste: 0.5 kg/d

Therefore, this list of ingredients and their quantities is the optimized animal feed composition 4 for that particular cow in that day. The cost corresponding to that feed ration is 4.06€.

The same steps are repeated for all the animals in the group 2, that is, all the cows in the pen, and the optimized group feed composition 1 to be fed in that day to the group 2 is, thus, the aggregated list of ingredients of all the optimized animal feed compositions 4 determined in step c).

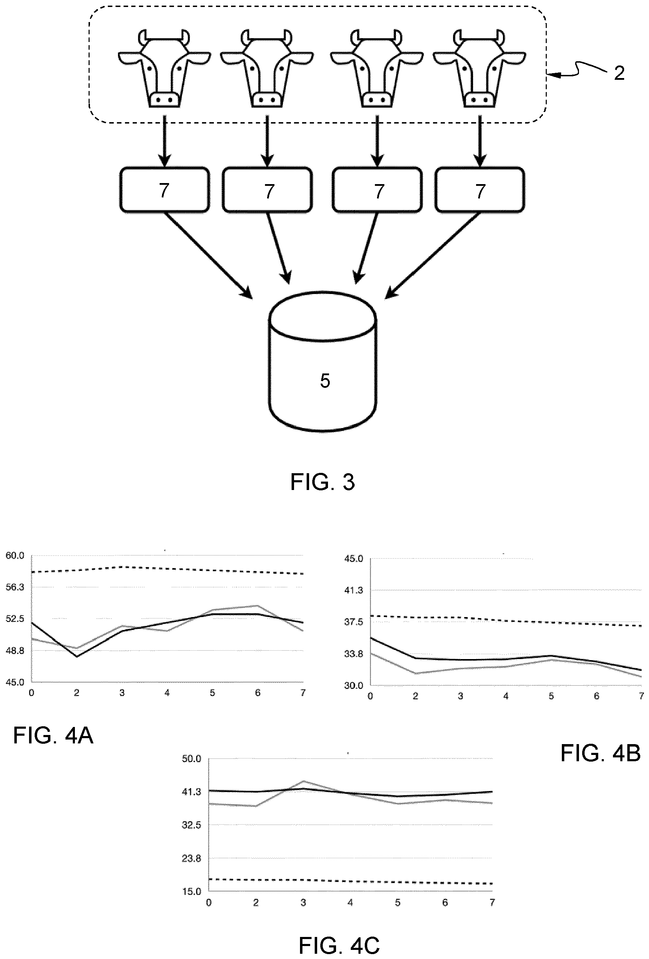

Fig. 3 shows further steps of the first embodiment of the method, aimed to the update of the local milk production model 5:
d) For each animal of the group 2, receiving a set of measures 7.
e) Updating the local milk prediction model 5 by incorporating said set of measures 7 for each animal of the group 2.
Steps e) and d) are repeated daily, and the measures are therefore corresponding to each day. Nevertheless, instead of single daily data, moving averages are used, using a rolling window of 30 days.

For the first embodiment, said set of measures 7 comprises measured data corresponding to the input data 51 and prediction of milk production 52 used by the local milk prediction model 5, in particular:
- A measure of consumed feed, said consumed feed being the feed composition that has been consumed by said animal in one day, i.e. the amounts of dry feed, crude protein and neutral detergent fibre, as well as the energy of the feed.
- The following measures of animal parameters: body weight, stage of lactation and lactation number.
- The following environmental parameters: minimum temperature, maximum temperature and average temperature.
- A measure of milk production, said milk production being the milk that has been produced by said animal in said day, said measure of milk production comprising said at least one milk parameter, i.e. the amount of milk production in kg.

However, in this embodiment, the measure of consumed feed for each animal of the group 2 is not a direct measure. On the contrary, is an estimated measure based in a measure of feed consumed by all the animals in the group 2. In particular, said estimated measures are obtained following the steps described herein. First, an expected consumption is obtained for each animal of the group 2. The expected consumption for each animal is obtained by using a prediction model configured to predict the feed intake of one animal based on its milk production and/or animal characteristics. Different prediction models can be envisaged, for example the NRC 2001, but is out of the scope of this document to describe in detail the characteristics of said models. The skilled person will select the most accurate model available in order to obtain the best estimated values possible, considering that the model has to depend on data that can be easily measured and that the computational requirements for the model must make it possible to use it locally. Second, a total expected consumption for the group is obtained, being the aggregation of each expected consumption for each of the animals of the group 2.

The expected consumptions are then used for weighting the consumption of each animal from the measured consumption of all the pen. In particular, a correction factor is obtained by dividing the measure of feed consumed by all the animals in the group 2 by the total expected consumption for the group 2. Finally, for each animal in the group 2, said correction factor is applied to the expected consumption for said animal.

A real-life example of application of the step d) of the first embodiment of the method is given herein. The following Table I summarizes the calculation of the correction factor for three consecutive days.

**Table I**

| Day | Head count | Measured pen intake, kg | Estimated feed intake, kg | Correction Factor |
|---|---|---|---|---|
| 1 | 23 | 615 | 557 | 1.10 |
| 2 | 23 | 591 | 561 | 1.05 |
| 3 | 24 | 586 | 586 | 1.00 |

The head count column corresponds to the number of animals in the group. Note that this number can be different from day to day, in the example, in the first and second days the group 2 has 23 animals, and in the third day, the group 2 has 24 animals.

The second column is the real measure of feed consumed by all the animals in the group 2. The third column is the total expected consumption for the group 2 which is obtained as the aggregation of each expected consumption for each of the animals of the group 2. The expected consumption in the exemplary table is calculated by a customized model, but other known models can be envisaged, for example the well-known NRC 2001. The last column is the ratio between the values in the second and third columns, thus being a correction factor.

Once calculated said correction factor it can be applied to the expected consumption of each animal thus obtaining the measure of consumed feed for each animal of the group 2. The following Table II summarizes the calculation for one particular animal in the group 2.

**Table II**

| Day | Head count | Correction Factor | Expected consumption, kg | Measure of consumed feed, kg |
|---|---|---|---|---|
| 1 | 23 | 1.10 | 24.58 | 27.04 |
| 2 | 23 | 1.05 | 26.82 | 28.16 |
| 3 | 24 | 1.00 | 26.78 | 26.78 |

The three first columns of Table II correspond to the equivalent columns in Table I. The expected consumption has been calculated using the customized model mentioned above. The last column is the measure of consumed feed obtained for said animal in step d) and used for updating the local milk production model 5 in step e). It corresponds to the multiplication of expected consumption by the correction factor.

As an example of the accuracy of the method, Fig. 4A, 4B and 4C show exemplary graphs comparing the milk production in kg for different consecutive days. Each figure represents a different animal in the pen. The black line corresponds to real measures of milk production and the grey line the prediction of milk 52 for the animal using the local milk prediction model 5 of the invention. As a reference, the dotted line has been calculated using the common known model described by the Wool equation describing an algebraic model of the lactation curve in cattle.

Fig. 5 shows a diagram of the complete system corresponding to the first embodiment. The figure depicts a federated system 200 having three local systems 100 and a central module 201. Each of the local systems 100 has a control module 101 comprising a local milk prediction model 5 and is configured for performing the method according to the embodiment described above. The central module 201 has a seed milk prediction model 6 that is a LSTM neural network. All the neural networks 5 and 6 have the same structure.

Each control module 101 is a computer running a software application having software instruction commands that, where are run by the computer execute the method described above. Other kind of hardware can also be envisaged, by the way of non-limiting examples, a plurality of computers, a portable device like a mobile phone or tablet, or a dedicated machine having computing capabilities. The communication between the central module 201 and each of the control modules 101 is done using World Wide Web communications, but other channels can be envisaged, for example, direct radio communications.

For the first embodiment, the method that is performed by the control modules 101 of local systems 100 further comprises the daily steps of:
- sending to the central module 201:
   - the set of measures 7; and
   - the internal status of the neural network nodes of the local milk prediction model 5;
- receiving from said central module 201 an updated milk prediction model that has been generated by the central module 201 using federated learning; and
- replacing the current local milk prediction model 5 with said updated milk prediction model.

In turn, each of the local systems 100 of the federated system 200 that is represented in Fig. 5 further comprises a mobile device 102 to assist in the creation of a feed mix that will be given to the animals. In each farm, the different ingredients to be used in the feed mix are stored in their respective storage locations. A truck moves from one storage location to another, loading different ingredients that are mixed together and given to the animals. The mobile device 102 moves together with the truck in order to assist in the creation of the feed mix, and is provided with a tracking system and a map having the storage locations of the ingredients, so when, the truck arrives to a storage location, the mobile device 102 it is able to determine the ingredient corresponding to said location. Other location means can be envisaged, for example, using RFID, NFC or beacons together with corresponding elements in the storage locations of the ingredients.

The mobile device 102 comprises display means, in particular a display screen, for displaying information to a user. It is also provided with communication means, in particular a wireless communication module, capable of using cell-phone data networks and/or WIFI networks. The mobile device 102 also has a weigh measure interface, for receiving measures of weights of ingredients.

In order to assist to the creation of the feed mix, the control module 101 sends to the mobile device 102 the optimized group feed composition 1, containing the list of ingredients and the weight of each ingredient.

The mobile device 102 receives said composition and, when the truck arrives to the storage location of each ingredient, it displays in the screen the quantity of the said ingredient that has to be used in the feed mix. The operator loads the quantity and the weight measurement interface receives the actual weight that has been loaded in the truck for said ingredient. The truck then moves to the next storage location, and the sequence is repeated until the feed mix has been completed. Using the communication means, the mobile device 102 sends the measures of the weight of each ingredient to the control module 101. At the end of the day, the control module 101 further receives a measure of the weight of the remaining feed that has not been consumed by the group 2 of animals. Using the measures of the weights of the ingredients provided by the mobile device 102 and removing from the amount the feed that has not been consumed, the control module 101 determines de feed consumed by all the animals in the group 2, which is latter used for determining the measure of the feed consumed by each animal according to the method described above.

Other embodiments of the method according to the invention are disclosed hereinafter. These embodiments share most of the features disclosed in the first embodiment above.

Therefore, only the differentiating features will be described in detail. For the sake of brevity, common features shared with the first embodiment disclosed above will not be described again hereinbelow.

In further embodiments, the milk parameters obtained by the local milk prediction model comprise not only the quantity of milk but also at least one of the following:
- quantity of milk;
- protein content of the milk;
- fat content of the milk;
- lactose content of the milk;
- calcium content of milk; and
- fatty acid profile of milk;
or combinations thereof. The target criterion 3 depends on the milk parameters available, therefore, the skilled person will correspondingly select the milk parameters to be used.

Related to the milk parameters, in some embodiments the target criterion 3 is obtaining a predetermined milk protein content value or range. Other possible target criteria 3 can be envisaged, for example, obtaining a predetermined milk fat content value or range, maximizing milk production benefit, or combinations thereof.

In another embodiment, the input data 51 of the local milk prediction model 5 further comprises at the humidity. Other embodiments, the input data comprises the hours of exposition to daylight.

For another embodiment, the expected consumption for each animal is obtained by using the optimized animal feed composition 4 for said animal, determined in step b).

## Claims

1. Method for determining an optimized group feed composition (1) to be fed in one day to a group (2) of animals that are bred to produce milk, in particular dairy cattle, sheep or goats, said group (2) having one or more animals;
**characterized in that** said method comprises the steps of:
a) determining a target criterion (3) for optimization, said target criterion (3) depending on at least one milk parameter;
b) for each animal of said group (2) of animals, determining an optimized animal feed composition (4) as the feed composition that optimizes said target criterion (3) using a local milk prediction model (5); and
c) determining said optimized group feed composition (1) to be fed to said group (2) of animals as the aggregation of said optimized animal feed compositions (4) for each of the animals of said group (2);
wherein said local milk prediction model (5) is configured for obtaining a prediction of milk production (52) of one animal in one day depending on input data (51); said input data (51) comprising a feed composition fed to said animal in said day; said prediction of milk production (52) comprising milk output data comprising said at least one milk parameter.

2. Method according to claim 1, **characterized in that** said optimized group feed composition (1) comprises a list of quantities of ingredients, a list of quantities of nutrients or a combination thereof.

3. Method according to any of the claims 1 or 2, **characterized in that** said at least one milk parameter comprises at least one of the following milk parameters:
- quantity of milk;
- protein content of the milk;
- fat content of the milk;
- lactose content of the milk;
- calcium content of milk; and
- fatty acid profile of milk;
or combinations thereof; and preferably wherein said target criterion (3) is one of:
- maximizing milk production;
- obtaining a predetermined milk protein content value or range;
- obtaining a predetermined milk fat content value or range; and
- maximizing milk production benefit;
or a combination thereof.

4. Method according to any of the claims 1 to 3, **characterized in that** said input data (51) of said local milk prediction model (5) further comprises at least one animal parameter, preferably, at least one of the list consisting in:
- body weight;
- stage of lactation;
- stage of pregnancy;
- lactation number; and
- genomic value of the animal;
and preferably it further comprises at least one environmental parameter, in particular at least one of temperature, humidity and hours of exposition to daylight.

5. Method according to any of the claims 1 to 4, **characterized in that** it further comprises the steps of:
d) for each animal of the group (2), receiving a set of measures (7), comprising:
- a measure of consumed feed, said consumed feed being the feed composition that has been consumed by said animal in one day;
- a measure of milk production, said milk production being the milk that has been produced by said animal in said day, said measure of milk production comprising said at least one milk parameter;
- preferably, at least a measure of an animal parameter; and
- preferably, at least a measure of an environmental parameter;
e) updating said local milk prediction model (5) by incorporating said set of measures (7) for each animal of the group (2);
wherein said steps d) and e) are preferably repeated daily.

6. Method according to claim 5, **characterized in that** said measures of consumed feed and said measures of milk production are, independently, obtained using respective moving averages.

7. Method according to any of the claims 5 or 6, **characterized in that**, for each animal of the group (2), said measure of consumed feed is an estimated measure based in a measure of feed consumed by all the animals in the group (2), wherein said estimated measures are preferably obtained by:
- obtaining an expected consumption for each animal of the group (2);
- obtaining a total expected consumption for the group (2), being the aggregation of each expected consumption for each of the animals of the group (2);
- dividing said measure of feed consumed by all the animals in the group (2) by said total expected consumption for the group (2), thus obtaining a correction factor; and
- for each animal in the group (2) applying said correction factor to said expected consumption for said animal.

8. Method according to claim 7, **characterized in that** said expected consumption for each animal is obtained by using a prediction model configured to predict the feed intake of one animal based on its milk production and/or animal characteristics.

9. Method according to any of claims 1 to 8, **characterized in that** said local milk prediction model (5) is a neural network, preferably a Long-Short Term Memory, LSTM, neural network.

10. Method according to claim 9, **characterised in that** it further comprises:
- sending to a central module:
- said set of measures (7); and
- the internal status of the neural network nodes of said local milk prediction model (5);
- receiving from said central module an updated milk prediction model; and
- replacing the current local milk prediction model (5) with said updated milk prediction model.

11. Computer program containing program instruction codes that, when executed by a computer, lead to said computer to perform the method according to any of the claims 1 to 10.

12. Local system (100) for determining optimized group feed compositions (1) to be fed to at least one group (2) of animals that are bred to produce milk, in particular dairy cattle, sheep or goats, each group (2) of animals having one or more animals, **characterized in that** said system comprises a control module (101) comprising a local milk prediction model (5) and is configured for performing the method according to any of the claims 1 to 10 for each group (2) of animals.

13. Local system (100) according to claim 12, **characterized in that** it further comprises a mobile device (102) to assist in the creation of a feed mix that will be given to one group (2) of animals of said at least one group (2) of animals, said mobile device (102) comprising:
- display means, for displaying information to a user;
- communication means, for communication with said control module (101); and
- weight measure receiving means, for receiving measures of weight of ingredients;
wherein said mobile device (102) is configured for:
- receiving from said control module (101) an optimized group feed composition (1) for one group (2) of animals of said at least one group (2) of animals, said optimized group feed composition (1) comprising a list of ingredients and the weight of each of said ingredients that has be added to a feed mix that will be given to said group (2) of animals;
- for each of said ingredients of said list of ingredients:
- displaying said weight of said ingredient using said display means; and
- receiving a measure of the weight of said ingredient that has been added to said feed mix;
- and, sending to said control module (101) said measures of the weight of each ingredient;
wherein said control module (101) is configured for determining a measure of feed consumed by all the animals in said group (2) according to said measures of the weight of each ingredient.

14. Local system (100) according to claim 13, **characterized in that** said control module (101) is further configured for obtaining a measure of weight of the remaining feed that has not been consumed by all the animals in said group (2), and wherein determining a measure of feed consumed by all the animals further includes said weight of the remaining feed.

15. Federated system (200) for determining optimized group feed compositions (1) to be fed to at least one group (2) of animals that are bred to produce milk, in particular dairy cattle, sheep or goats, each group (2) of animals having one or more animals, **characterized in that** said system comprises:
- at least one local system (100) according to any of the claims 12 to 14; and
- a central module (201), comprising a seed milk prediction model (6);
wherein said seed milk prediction model (6) and the local milk prediction models (5) of said at least one local system (100) are all neural networks, preferably LSTM neural networks, having the same node structure; each neural network having an internal status;
said central module (201) configured for receiving from each control module (101) corresponding to each of said at least one local system (100):
- a set of measures (7); and
- said internal status of the neural network of said local milk prediction model (5);
said set of measures (7) comprising, for each animal:
- a measure of consumed feed, said consumed feed being the feed composition that has been consumed by said animal in one day;
- a measure of milk production, said milk production being the milk that has been produced by said animal in said day, said measure of milk production comprising said at least one milk parameter;
- preferably, at least a measure of an animal parameter; and
- preferably, at least a measure of an environmental parameter;
said central module (201) further configured for generating, using federated learning, an updated milk prediction model for each of said at least one local system (100); and sending each updated milk prediction model to the corresponding control module (101) of each local system (100).
